# EUROPEAN PATENT APPLICATION

(11) **EP 2 554 995 A1**
(43) Date of publication of application: **06.02.2013**
(21) Application number: 11176486.6
(22) Date of filing: 03.08.2011
(51) Int. Cl.: G01N 33/68

(54) **Troponin based rule in and rule out algorithm of myocardial infarction**

(71) Applicant: Roche Diagnostics GmbH, 68305 Mannheim (DE); F. Hoffmann-La Roche AG, 4070 Basel (CH)
(72) Inventor: Jarausch, Jochen, 82362 Weilheim (DE); Menassanch-Volker, Sylvie, 6340 Baar (CH); Müller, Christian, 4056 Basel (CH); Reichlin, Tobias, 6043 Adligenswil (CH); Verhagen-Kamerbeek, Wilma, 4148 Pfeffingen (CH); Weiser, Silvia, 82061 Neuried (DE); Zaugg, Christian, 4310 Rheinfelden (CH)
(74) Representative: Dick, Alexander

(57) **Abstract**

The present invention relates to a method for diagnosing myocardial infarction in a subject presenting with chest pain. The method is based on the determination the amount of a cardiac troponin in a first sample from the subject obtained at presentation to a physician, and in a second sample obtained within one hour after the first sample. Moreover, the present invention envisages a method for ruling in myocardial infarction and a method for ruling out myocardial infarction. The said methods are also based on the determination the amount of a cardiac troponin in a first sample from the subject obtained at presentation to a physician, and in a second sample obtained within one hour after the first sample.

## Description

Acute myocardial infarction is a major cause of death and disability. Approximately 15 million patients per year in the United States and Europe present to the emergency department with chest pain or other symptoms suggestive of acute myocardial infarction. Rapid identification of acute myocardial infarction is critical for the initiation of effective evidence-based medical treatment and management. Electrocardiography (ECG) by itself is often insufficient to diagnose an acute coronary syndrome or acute myocardial infarction, since ST-segment deviation may be observed in other conditions, such as acute pericarditis, left ventricular hypertrophy. Cardiac troponins, which are structural proteins unique to the heart, are sensitive and specific biochemical markers of myocardial damage (see e.g., Reichlin et al., N Engl. J Med 2009; 361: 858-67; Anderson et al. ACC/AHA 2007 guidelines for the management of patients with unstable angina/non ST-elevation myocardial infarction: a report of the American College of Cardiology/American Heart Association Task Force on Practice Guidelines (Circulation 2007;116:e148-e304, Bassand et al., Eur Heart J 2007;28:1598-1660; Thygesen et al. Circulation 2007;116:2634-2653).

Cardiac troponins are very helpful in clinical practice for identifying patients with acute coronary syndromes who are at high risk and for selecting patients who will benefit from an early invasive strategy and glycoprotein IIb/IIIa blockade. In addition, cardiac troponin levels, as measured by fully automated standard assays such as the current fourth-generation Roche Troponin T, are superior to all other clinically available biomarkers, including myoglobin, the MB fraction of creatine kinase (CK-MB), myeloperoxidase, and heart fatty acid-binding protein, for the diagnosis of acute myocardial infarction (see, e.g. McCann et al., Eur Heart J 2008;29:2843-2850).

The major limitation of standard cardiac troponin assays is their low sensitivity at the time of a patient's presentation, owing to a delayed increase in circulating levels of cardiac troponins. The diagnosis of acute myocardial infarction consequently requires prolonged monitoring over a period of 6 to 12 hours and serial blood sampling. A delay in confirming a diagnosis of acute myocardial infarction may increase the risk of complications associated with the condition and a delay in ruling out the diagnosis contributes to overcrowding in the emergency department, with the associated costs.

Reichlin et al. (loc. cit) examined the diagnostic accuracy of recently introduced high sensitive cardiac troponin assays sensitive from four different manufacturers performed on blood samples obtained from patients who presented with symptoms suggestive of acute myocardial infarction. They showed that recently introduced high sensitive cardiac troponin assays can improve the diagnosis of myocardial infarction, particularly in patients with a recent onset of chest pain.

On a Congress of the European Society of Cardiology held in Stockholm from August 28 to September 1, 2010 Reichlin et al. presented a study in which hs-cTnT levels were determined in patients with suspected myocardial infarction. The levels of TnT were determined in samples obtained at presentation and after 1, 2, 3 and 6 hours. Reichlin et al. conclude that absolute but not relative changes of hs-cTnT have a very high diagnostic accuracy and should be used in conjunction with baseline values for the diagnosis of myocardial infarction. In patients with a baseline hs-cTnT level ≤12.5 ng/l and with a change in hs-cTnT ≤2 ng/l within the first hour, AMI was ruled out with a sensitivity and negative predictive value of 100% at a specificity of 73% and a positive predictive value of 47%. According to Reichlin et al., the combination of baseline values and early changes within the first hour would seem to allow a rapid and reliable rule out of AMI, see Reichlin et al. European Heart Journal (2010) 31 (Abstract Supplement), 51, Abstract P497).

This was confirmed in a further study carried out by Reichlin et al (Reichlin et al., Circulation. 2011;124:136-145). In particular, it was also shown that absolute changes of Troponin levels have a significantly higher diagnostic accuracy for AMI than relative changes.

Although the cTnT-hs assay has been shown to improve the early diagnosis of AMI, it is currently unknown how to best use it in clinical practice. Specifically, the increased sensitivity of the cTnT-hs assay is at the expenses of a reduced specificity leading to frequent detection of non-acute coronary syndrome related increases in cTnT concentrations. As a consequence, according to guidelines, serial measurements with a rise and/or fall are required to diagnose AMI and distinguish from elevated values due to causes other than acute coronary syndrome.

Thus, there is still a need to refine the diagnosis of myocardial infarction in patients presenting with chest pain.

The technical problem underlying the present invention can be seen as the provision of means and methods for reliably and efficiently diagnosing myocardial infarction in a subject exhibiting chest pain. The technical problem is solved by the embodiments characterized in the claims and herein below.

Advantageously, it was shown in the context of the present invention that the combination of baseline hs-cTnT levels with absolute changes within the first hour after presentation was superior to both baseline (p<0.001) and 1h levels (p=0.03) for the diagnosis of AMI (area under the curve 0.98, 95% CI 0.97-0.99). The hs-cTnT algorithm incorporating baseline values as well as absolute changes within the first hour allowed for a rule-out of AMI in 74% of non-AMI patients with a sensitivity and negative predictive value of 100%. On the other hand side, 87% of all AMI patients could be ruled-in within 1 hour with a specificity of 97% and a positive predictive value of 81%.

Thus, using an algorithm incorporating Troponin baseline values and absolute changes within the first hour, a safe rule-out as well as an accurate rule-in of AMI can be performed within 1 hour in 80% of all chest pain patients. If applied, the algorithm will obviate the need for prolonged monitoring and serial blood sampling in the majority of chest pain patients.

Accordingly, the present invention relates to a method for diagnosing myocardial infarction in a subject presenting with chest pain, comprising the steps of:
a) determining the amount of a cardiac troponin in a first sample from the subject obtained at presentation to a physician, and
b) determining the amount of a cardiac troponin in a second sample from said subject obtained within one hour after the first sample,

(A) wherein the subject does not suffer from myocardial infarction,
   (i) if the subject is older than 75 years and the amount of the cardiac troponin in the second sample is less than 25 ng/l, or
   (ii) if the subject is younger than 75 years and if the amount of the cardiac Troponin in the first sample is less than 12 ng/l, and if the difference between the amount of the cardiac troponin in the second sample and the amount in first sample is less than 3 ng/l,
(B) wherein the subject suffers from myocardial infarction,
   (i) if the amount of the cardiac Troponin in the first sample is less than 12ng/l and if the difference between the amount of the cardiac Troponin in the second sample and the first sample is at least 15 ng/l,
   (ii) if the amount of the cardiac troponin in the first sample is between 12 and less than 60 ng/l (and, thus, is in particular larger than or equal to 12 ng/l and less than 60 ng/l) and if the difference between amount of the cardiac troponin in the second sample and the amount of the cardiac troponin in the first sample is at least 15 ng/l, or
   (iii) if the amount of the cardiac Troponin in the first sample is at least 60 ng/l.

In a preferred embodiment the method, preferably, comprises the further steps:
c) assessing the difference between the amount of the cardiac troponin in the second sample and the amount of said cardiac troponin the first sample, and/or
d) comparing the amount of the cardiac troponin in the first sample and the amount in the second sample to reference amounts,

Preferably, it is diagnosed whether the subject presenting with chest pain suffers from myocardial infarction, or not, by carrying out the further step of e) diagnosing whether said subject suffers from myocardial infarction, or not, based on the result of the assessment carried out in step c) and the comparison carried out in step d).

The method of the present invention, preferably, is an ex vivo method. Moreover, it may comprise steps in addition to those explicitly mentioned above. For example, further steps may relate to sample pre-treatments or evaluation of the results obtained by the method. The method may be carried out manually or assisted by automation. Preferably, step (a), (b) and, if carried out, step (c) may in total or in part be assisted by automation, e.g., by a suitable robotic and sensory equipment for the determination in steps (a) and/or (b), or a computer-implemented assessment or comparison in steps (c) and/or (d) and/or diagnosis based on said assessment and comparison. More preferably, the method is carried out entirely in an automated manner. In such a case, the diagnostic result which is established in step is generated in a suitable output format so that it can be used as an aid for establishing the final clinical diagnosis by, e.g., a medical practitioner.

The term "diagnosing" as used herein means assessing whether a subject as referred to in accordance with the method of the present invention suffers from myocardial infarction or not. As will be understood by those skilled in the art, such an assessment is usually not intended to be correct for 100% of the subjects to be diagnosed. The term, however, requires that the assessment is correct for a statistically significant portion of the subjects (e.g. a cohort in a cohort study). Thus, the method of the present invention, however, at least provides an aid for establishing a final clinical diagnosis. Whether a portion is statistically significant, can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, 0.001 or 0.0001.

In the context of the method of the present invention it shall be assessed whether a subject presenting with chest pain (in particular, a subject exhibiting chest pain) suffers from myocardial infarction or not. The term "myocardial infarction" (abbreviation MI) is well known in the art. Preferably, the term refers to acute myocardial infarction ("AMI"). Preferably, the term includes ST-elevation MI (STEMI) and non-ST-elevated MI (NSTEMI). Preferably, a subject suffers from myocardial infarction if the subject fulfills the criteria for MI as set forth in Thygesen K, Alpert JS, White HD. Universal definition of myocardial infarction. Eur Heart J 2007;28:2525-38 which herewith is incorporated by reference with respect to its entire disclosure content.

Accordingly, a subject, preferably, suffers from myocardial infarction if the subject suffers from myocardial necrosis and if there is evidence of myocardial ischemia. In particular, a subject suffers from myocardial infarction if the levels of cardiac troponin are above the 99th percentile of the reference limit (of a healthy population) together with evidence of myocardial ischemia (symptoms, ECG changes or imaging results). Preferably, the definition requires a cardiac troponin assay with an imprecision (coefficient of variation) at the 99th percentile less than or equal to 10 %. Also preferably, the subject suffers from necrosis if at least one amount (value) of the cardiac troponin above the 99th percentile in one or more samples obtained at presentation and within six to nine hours after presentation from the subject (in particular with an imprecision of less than 10%) together with a significant rise and/or fall of the amount of the cardiac troponin. A significant rise or fall, preferably, is a change, i.e. a rise or fall of the amount of cardiac Troponin of at least 30% of the 99^{th} percentile within 6 to 9 hours after presentation to a physician, in particular after the first sample has been obtained. Whether there is evidence of ischemia can be determined by the skilled person without further ado. Preferably, there is evidence of ischemia, if there are clinical symptoms of ischaemia. Symptoms of ischemia, preferably, include various combinations of chest, upper extremity, jaw, or epigastric discomfort with exertion or at rest. Often, the discomfort is diffuse, not localized, not positional, not affected by movement of the region, and it may be accompanied by dyspnoea, diaphoresis, nausea, or syncope. Moreover, there is evidence of ischemia if there are electrocardiogram (ECG) changes indicative of new ischemia (new ST-T changes or new left bundle branch block), or if there are pathological Q wave changes in the ECG.

The term "subject" as used herein relates to animals, preferably mammals, and, more preferably, humans, preferably men or women. The subject shall present with symptoms of chest pain. In particular the subject shall present with chest pain symptoms such as acute chest pain and angina pectoris, preferably with chest pain symptoms with an onset or peak within the last 12 hours, in particular with chest pain symptoms with an onset or peak within the last 12 hours before presentation to a physician, or, more preferably, before the first sample is obtained. The symptoms of chest pain may persist or may not persist at presentation to a physician. Accordingly, the subject presenting with chest pain may still exhibit chest pain at presentation to a physician, or not.

Preferably, the term "chest pain" includes any pain that occurs in the area between the neck and the bottom of the rib cage. The chest pain may have several causes. In principle, any part of the chest can be the cause of the pain including the heart, lungs, esophagus, muscle, bone, and skin. The terms "chest pain", "acute chest pain" and "angina pectoris" as used herein are generally known to the skilled physician.

In particular, the chest pain may be caused by myocardial infarction (MI), by unstable angina (UA), by cardiac disease (or disorder) other than coronary artery disease, or by non-cardiac causes. Prefferred cardiac diseases (or disorders) other than coronary artery disease that may cause chest pain include myocarditis, pericarditis, takotsubo cardiomyopathy, cardiac arrhythmia, hypertensive crisis, and heart failure. Preferred non-cardiac causes of chest pain include musculosceletal chest pain, pleuritis, pneumonia, pneumothorax, gastritis, gastro esophageal reflux disease, and anxiety disorder. Preferably, the chest pain has a duration of at least 20 minutes. It is also envisaged that the chest pain has a duration of several hours, but this may vary interindividually. Accordingly, the chest pain pain may have a duration of at least one hour, or of at least two hours, or of at least five hours. As set forth elsewhere herein, the chest pain may persist or may not persist at presentation.

Preferably, the chest pain did not occur in connection with physical exercise. More preferably, the chest pain did not occur in connection with a high level of physical exercise. Accordingly, the subject, preferably, did not carry out physical exercise before the onset of symptoms of chest pain, or did carry out only low levels of physical exercise before the onset of symptoms of chest pain.

The subject of the present inventions, preferably, does not exhibit impaired renal function. How to assess whether a subject exhibits impaired renal function is well known in the art. Particularly, renal function can be assessed by means of the glomerular filtration rate (GFR). For example, the GFR may be calculated by the Cockgroft-Gault or the MDRD formula (Levey 1999, Annals of Internal Medicine, 461-470). GFR is the volume of fluid filtered from the renal glomerular capillaries into the Bowman's capsule per unit time. Clinically, this is often used to determine renal function. The GFR was originally estimated (the GFR can never be determined, all calculations derived from formulas such as the Cockgroft Gault formula or the MDRD formula deliver only estimates and not the "real" GFR) by injecting inulin into the plasma. Since inulin is not reabsorbed by the kidney after glomerular filtration, its rate of excretion is directly proportional to the rate of filtration of water and solutes across the glomerular filter. In clinical practice however, creatinine clearance is used to measure GFR. Creatinine is an endogenous molecule, synthesized in the body, which is freely filtered by the glomerulus (but also secreted by the renal tubules in very small amounts). Creatinine clearance (CrCl) is therefore a close approximation of the GFR. The GFR is typically recorded in milliliters per minute (mL/min). The normal range of GFR for males is 97 to 137 mL/min, the normal range of GFR for females is 88 to 128 ml/min. Thus, it is particularly contemplated that the GFR of the subject as referred to herein is within this range. Moreover, the subject as referred to in the context of the method present invention, preferably, has a blood creatinine level (in particular a serum creatinine level) of lower than 0.9 mg/dl, more preferably of lower than 1.1 mg/dl and most preferably of lower than 1.3 mg/dl.

It is particularly envisaged that the subject as referred herein in the context of the methods of the present invention does not suffer from terminal kidney failure. Preferably, the subject to be tested does not suffer from terminal kidney failure requiring dialysis.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma, urine or serum and, even more preferably, blood, plasma or serum. The most preferred sample is a blood sample, in particular a plasma or serum sample. Both serum and plasma samples have been successfully used in the context of the studies underlying the present invention. Tissue or organ samples may be obtained from any tissue or organ by, e.g., biopsy. Separated cells may be obtained from the body fluids or the tissues or organs by separating techniques such as centrifugation or cell sorting.

The first sample shall be obtained from the subject to be tested at presentation to a physician, in particular at the first presentation to a physician or to medical personnel after the onset of chest pain. Preferably, the phrase "presentation to a physician" refers to the presentation to the emergency department, in particular to the presentation to a physician at the emergency room or department, to the presentation to a paramedic, to the presentation to an emergency physician, or to the arrival of an ambulance vehicle, or to the arrival of the subject in the hospital. Preferably, the phrase "presentation to a physician" refers to the presentation to the emergency department, in particular to the first presentation to a physician at the emergency department.

Preferably, a sample is deemed to be obtained at presentation to a physician (in particular to the emergency department), if the sample is obtained not more than 90, or not more than 60 minutes, or not more than 40 minutes after presentation to the physician (in particular to the emergency department). More preferably, a sample is deemed to be obtained at presentation to a physician, if the sample is obtained not more than 30 or 15 minutes after presentation to a physician. Most preferably, a sample is deemed to be obtained at presentation to a physician, if the sample is obtained not more than 30 minutes after presentation to a physician.

In addition, the first sample, preferably, is obtained within 24 hours after the onset or peak of chest pain (and, thus, not later than 24 hours after the onset or peak of chest pain). More preferably, the first sample is obtained within 12 hours after the onset or peak of chest pain (and, thus, not later than 12 hours after the onset or peak of chest pain). Even more preferably, the first sample is obtained within 6 hours after the onset or peak of chest pain (and, thus, not later than 6 hours after the onset or peak of chest pain). It is further contemplated that the first sample is obtained within 3 or 4 hours after the onset or peak of chest pain (and, thus, not later than 3 or 4 hours after the onset or peak of chest pain). The aforementioned periods are preferably, drawn to the onset of chest pain.

As set forth above, the first sample shall be obtained within certain periods after the onset or peak of chest pain. Preferably, however, the first sample is not obtained too early after the onset of chest pain. Thus, it is preferred that the first sample is not obtained within 1 hour after the onset of chest pain (and, thus, the sample is, preferably, obtained not earlier than 1 hour after the onset of chest pain). It is further particularly preferred that the first sample is not obtained within 2 hours after the onset of chest pain (and, thus, the sample is, preferably, obtained not earlier than 2 hours after the onset of chest pain). Also, it is preferred that the first sample is not obtained within 3 hours after the onset of chest pain (and, thus, the sample is, preferably, obtained not earlier than 3 hours after the onset of chest pain).

In the context of the embodiments of the present invention, the first sample shall be obtained at presentation to a physician. Alternatively (and, thus, independently from the presentation at a physician), it is particularly envisaged that the first sample, preferably, is obtained within 24 hours after the onset or peak of chest pain (and, thus, not later than 24 hours after the onset or peak of chest pain). More preferably, the first sample is obtained within 12 hours after the onset or peak of chest pain (and, thus, not later than 12 hours after the onset or peak of chest pain). Even more preferably, the first sample is obtained within 6 hours after the onset or peak of chest pain (and, thus, not later than 6 hours after the onset or peak of chest pain). It is further contemplated that the first sample is obtained within 3 hours after the onset or peak of chest pain (and, thus, not later than 3 hours after the onset or peak of chest pain).

The second sample, as used herein is, preferably, obtained within one hour after the first sample. A sample shall be deemed to have been obtained within one hour after the first sample if is has been obtained between 30 to 90 minutes after the first sample (i.e. not earlier than 40 minutes, but not later than 80 minutes after the first sample). More preferably, the sample that has been obtained not later than one hour after the first sample has been obtained between 40 to 80 minutes or between 50 to 70 minutes after the first sample. Even more preferably, said sample has been obtained between 55 to 65, or 57 to 63 minutes after the first sample. Also preferably, said sample has been obtained 60 minutes after the first sample. In the most preferred embodiment, the second sample has been obtained between 45 to 75 minutes after the first sample (i.e. not earlier than 45 minutes, but not later than 75 minutes after the first sample).

The term "cardiac Troponin", preferably, refers to all Troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac Troponin refers to Troponin T and/or Troponin I, and, most preferably, to Troponin T. It is to be understood that isoforms of Troponins may be determined in the method of the present invention together, i.e. simultaneously or sequentially, or individually, i.e. without determining the other isoform at all. Amino acid sequences for human Troponin T and human Troponin I are disclosed in Anderson, loc cit and Ferrieres 1998, Clinical Chemistry, 44: 487-493.

The term "cardiac Troponin" encompasses also variants of the aforementioned specific Troponins, i.e., preferably, of Troponin I, and more preferably, of Troponin T. Such variants have at least the same essential biological and immunological properties as the specific cardiac Troponins. In particular, they share the same essential biological and immunological properties if they are detectable by the same specific assays referred to in this specification, e.g., by ELISA Assays using polyclonal or monoclonal antibodies specifically recognizing the said cardiac Troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, at least 60%, at least 70%, at least 80%, at least 85%, at least 90%, at least 92%, at least 95%, at least 97%, at least 98%, or at least 99% identical with the amino sequence of the specific Troponin, preferably over the entire length. Variants may be allelic variants or any other species specific homologs, paralogs, or orthologs. Moreover, the variants referred to herein include fragments of the specific cardiac Troponins or the aforementioned types of variants as long as these fragments have the essential immunological and biological properties as referred to above. Preferably, the cardiac troponin variants have immunological properties (i.e. epitope composition) comparable to those of human troponin T or troponin I. Thus, the variants shall be recognizable by the aforementioned means or ligands used for determination of the concentration of the cardiac troponins. Thus, the variants shall be recognizable by the aforementioned means or ligands used for determination of the concentration of the cardiac troponins. Such fragments may be, e.g., degradation products of the Troponins. Further included are variants which differ due to posttranslational modifications such as phosphorylation or myristylation. Preferably the biological property of troponin I and its variants is the ability to inhibit actomyosin ATPase or to inhibit angiogenesis in vivo and in vitro, which may e.g. be detected based on the assay described by Moses et al. 1999 PNAS USA 96 (6): 2645-2650). Preferably the biological property of troponin T and its variant is the ability to form a complex with troponin C and I, to bind calcium ions or to bind to tropomyosin, preferably if present as a complex oftroponin C, I and T or a complex formed by troponin C, troponin I and a variant of troponin T. It is known that low concentrations of circulating cardiac troponin may be detected in subjects at various conditions, but further studies are required to understand their respective role and rate (Masson et al., Curr Heart Fail Rep (2010) 7:15-21).

Determining the amount of a peptide or polypeptide may, preferably, comprises the steps of (a) contacting the peptide with a specific detection agent, (b) (optionally) removing non-bound detection agent, (c) measuring the amount of bound detection agent. The bound detection agent will generate an intensity signal. Binding according to the present invention includes both covalent and noncovalent binding. A detection agent according to the pre-sent invention can be any compound, e.g., a peptide, polypeptide, nucleic acid, or small molecule, binding to the peptide or polypeptide described herein. Preferred detection agents include antibodies, nucleic acids, peptides or polypeptides such as receptors or bind-ing partners for the peptide or polypeptide and fragments thereof comprising the binding domains for the peptides, and aptamers, e.g. nucleic acid or peptide aptamers. Methods to prepare such detection agents are well-known in the art. For example, identification and production of suitable antibodies or aptamers is also offered by commercial suppliers. The person skilled in the art is familiar with methods to develop derivatives of such detection agents with higher affinity or specificity. For example, random mutations can be intro-duced into the nucleic acids, peptides or polypeptides. These derivatives can then be tested for binding according to screening procedures known in the art, e.g. phage display. Anti-bodies as referred to herein include both polyclonal and monoclonal antibodies, as well as fragments thereof, such as Fv, Fab and F(ab)2 fragments that are capable of binding antigen or hapten. The present invention also includes single chain antibodies as well as genetically re-engineered chimeric human/mouse antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the detection agent binds specifically to the peptide or polypeptide, preferably to cardiac troponin T or cardiac troponin I, preferably to human cardiac troponin T or human cardiac troponin I. Specific binding according to the present invention means that the detection agent should not bind substantially to ("cross-react" with) another peptide, polypeptide or substance present in the sample to be analyzed. In particular, the detection agent, in particular the antibody, shall specifically bind to human cardiac Troponin T or I, but shall not bind to the isoforms of Troponin T and I, respectively, from the skeletal. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the detection agent can be measured by any method known in the art. Preferably, said method is semi-quantitative or quantitative. Further suitable techniques for the determination of a polypeptide or peptide are described in the following.

First, binding of a detection agent may be measured directly, e.g. by NMR or surface plasmon resonance. Second, if the detection agent also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the detection agent may exhibit enzymatic properties itself and the "detection agent/peptide or polypeptide" complex or the detection agent which was bound by the peptide or polypeptide, respectively, may be incubated with a suitable substrate allowing detection by the generation of an intensity signal. For measurement of enzymatic reaction products, preferably the amount of substrate is saturating. The substrate may also be labeled with a detectable label prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured. Third, the detection agent may be coupled covalently or non-covalently to a label allowing detection and measurement of the detection agent. Labeling may be done by direct or indirect methods. The detection agent or substrate may also be "tagged" with one or more tags as known in the art. Such tags may then be targets for higher order detection agents. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, rutheni-um, enzymatically active labels, radioactive labels, magnetic labels ("e.g. magnetic beads", including paramagnetic and superparamagnetic labels), and fluorescent labels. Enzymati-cally active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue te-trazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include 35S, 125I, 32P, 33P and the like. A radioactive label can be detected by any method known and appropriate, e.g. a light-sensitive film or a phosphor imager. Suitable measurement methods according the present invention also include precipitation (particularly immunoprecipitation), electro-chemiluminescence (electro-generated chemiluminescence), RIA (radioimmunoassay), ELISA (enzyme-linked immunosorbent assay), sandwich enzyme immune tests, electro-chemiluminescence sandwich immunoassays (ECLIA), dissociation-enhanced lanthanide fluoro immuno assay (DELFIA), scintillation proximity assay (SPA), turbidimetry, nephe-lometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The amount of a peptide or polypeptide may be, also preferably, determined as follows: (a) contacting a solid support comprising a detection agent for the peptide or polypeptide as specified above with a sample comprising the peptide or polypeptide and (b) measuring the amount peptide or polypeptide which is bound to the support. The detection agent, prefera-bly chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The detection agent may be bound to many different carriers. Examples of well-known carriers include glass, polystyrene, polyvinyl chloride, polypropylene, polyethylene, polycarbonate, dextran, ny-lon, amyloses, natural and modified celluloses, polyacrylamides, agaroses, and magnetite. The nature of the carrier can be either soluble or insoluble for the purposes of the invention. Suitable methods for fixing/immobilizing said detection agent are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different mi-crobeads or microspheres, possibly labelled, carrying different detection agents. Methods of producing such arrays, for example based on solid-phase chemistry and photo-labile protective groups, are generally known (US 5,744,305).

In the context of the studies of the present invention preferred reference amounts as well as preferred differences of the amount of the cardiac troponin in the second sample as compared to the first sample were developed in a derivation sample of 444 patients selected by stratified randomization according to "presence vs. absence of AMI" and "age ≥75 years vs. < 75 years". Optimal thresholds for rule-in were obtained by minimizing the sum of false positive and false negative decisions. Optimal thresholds for rule-out were selected to allow for a 100% sensitivity and negative predictive value (NPV). The algorithm was then tested prospectively in a validation data set of the remaining 443 subjects.

The reference amounts as set forth herein as well as the differences of the amount of the cardiac troponin between the first and the second sample were established using Roche's electrochemiluminescence ELISA sandwich test Elecsys Troponin T hs (high sensitive) assay, as specified in the examples, under "Methods". The test is described in Giannitsis et al. (Giannitsis E, Kurz K, Hallermayer K, Jarausch J, Jaffe AS, Katus HA. Analytical Validation of a High-Sensitivity Cardiac Troponin T Assay. Clin Chem. 56(2) 254-261, 2009) which herewith is incorporated by reference with respect to its entire disclosure content. Preferably, the assay described by Giannitsis et al. is used for the determination of the cardiac Troponin, in particular for Troponin T, in the context of the methods of the present invention.

In particular, Giannitsis et al. describe an assay based on the Elecsys®/cobas e™ cTnT fourth-generation assay from Roche Diagnostics) on the Elecsys 2010/cobas e 411 and Modular® Analytics E170/cobas e 601 immunoanalyzers (Roche Diagnostics). The assay uses fragment antigen-binding (FAB) fragments of two cTnT specific mouse monoclonal antibodies in a sandwich format. The antibodies recognize epitopes located in the central part of the cTnT molecule (amino acid positions 125-131 and 135-147, respectively). Detection is based on an electrochemiluminescence immunoassay (ECLIA), using a Tris(bipyridyl)-ruthenium(II) complex as label. The assay described by Giannitsis et al. is a hs-cTnT assay is a modification of the fourth-generation cTnT assay. The biotinylated capture antibody remained unchanged. However, the detection antibody was genetically reengineered, replacing the constant C1 region in the monoclonal mouse FAB fragment with a human IgG C1 region, leading to a mouse-human chimeric detection antibody.

It is well known in the art that amounts of a cardiac Troponin as determined with a specific assay may show some variance. Therefore, the reference amounts and the amounts for the differences (between the first and the second amount) given herein are, preferably, approximate amounts. Accordingly, the reference amounts and the amounts for the differences as set forth herein, preferably, may differ by 30% (+/-30%). More preferably, the amounts may differ by 20% (+/-20%). Even more preferably, the amounts may differ by 10% (+/-10%). Most preferably, the amounts may differ by 5% (+/-5%). It is also envisaged that the amounts may differ by 2 or 1%. Preferably, the amounts are the exact amounts.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be com-pared to values corresponding to suitable references which are stored in a database by a computer program. The computer program may further evaluate the result of the compari-son, i.e. automatically provides the desired assessment in a suitable output format, i.e. the diagnostic result. The said diagnostic result may, preferably, serve as an aid for establishing the final clinical diagnosis by, e.g., a medical practitioner.

In step c) of the method of the present invention, the difference between the amount of the cardiac troponin in the second sample and the amount of said cardiac troponin in the first sample shall be assessed.

The difference between the amount of a cardiac Troponin in the second sample as compared to the amount in the first sample can be an increase or decrease between the amount of a cardiac Troponin in the second sample as compared to the amount in the first sample. Accordingly, the term "difference" includes an increase of the amount of the cardiac troponin in the second sample as compared to the amount of the cardiac troponin in the first sample, or a decrease of the amount of the cardiac troponin in the second sample as compared to the amount of the cardiac troponin in the first sample. Thus, the term "difference", preferably, means a decrease or an increase of the amount of the cardiac troponin. Accordingly, the amount of the cardiac Troponin in the second sample differs from the amount in the first sample by a certain amount, if the amount of the cardiac Troponin in increased or decreased by the certain amount in the second sample as compared to the first sample.

For example, if the difference between the amount of the cardiac Troponin in the second sample and the amount in the first sample shall be at least 15 ng/l, the criteria is fulfilled if either the increase or decrease between the amount in the second sample as compared to the first sample is at least 15 ng/l. Thus, if the amount in the second sample is decreased, the decrease shall be at least 15 ng/l. If the amount in the second sample is increased, the increase shall be at least 15 ng/l.

For example, if the difference between the amount of the cardiac Troponin in the second sample and the amount in the first sample shall be at least 60 ng/l, the criteria is fulfilled if the increase or the decrease between the amount in the second sample as compared to the first sample is at least 60 ng/l. Thus, if the amount in the second sample is decreased, the decrease shall be at least 60 ng/l. If the amount in the second sample is increased, the increase shall be at least 60 ng/l.

For example, if the difference between the amount of the cardiac troponin in the second sample and the amount of the cardiac troponin in the first sample shall between 3 ng/l and less than 15 ng/l, the criteria is fulfilled, if the increase or decrease between the amount in the second sample as compared to the first sample is between 3 ng/l and less than 15 ng/l, i.e. equal to or larger than than 3 ng/l, or lower than 15 ng/l. Thus, if the amount is decreased, the decrease shall be between 3 ng/l and less than 15 ng/l. If the amount is increased, the increase shall be between 3 ng/l and less than 15 ng/l.

E.g., if the difference between the amount of the cardiac troponin in the second sample and the amount in first sample shall less than 3 ng/l, the criteria is fulfilled, if the increase or the decrease between the amount in the second sample as compared to the first sample is less than 3 ng/l. Thus, if the amount in the second sample is decreased, the decrease in the second sample shall be less than 3 ng/l. If the amount in the second sample is increased, the increase in the second sample shall be less than 3 ng/l.

The term "reference amount(s)" as used herein refers to an amount (amounts) which allows for allocation of a subject into (A) the group of subjects not suffering from (or unlikely to suffer from) myocardial infarction (rule out), or into (B) the group of subj ects suffering from (or likely to suffer from) myocardial infarction (rule in), or, optionally, into (C) a group of subjects requiring further monitoring in order to diagnose myocardial infarction (herein also referred to as "intermediate zone").

Reference amounts can, in principle, be calculated for a cohort of subjects as specified above based on the average or mean values for a given biomarker by applying standard methods of statistics. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity versus specificity pairs resulting from continuously varying the decision threshold over the entire range of data observed. The clinical performance of a diagnostic method depends on its accuracy, i.e. its ability to correctly allocate subjects to a certain diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects belonging to (A) the group of subjects not suffering from myocardial infarction, or to (B) the group of subjects suffering from myocardial infarction, or, optionally, to (C) a group of subjects requiring further monitoring in order to diagnose myocardial infarction, preferably, by establishing a ROC for said cohort as described above and deriving a threshold amount therefrom. Dependent on a desired sensitivity and specificity for a diagnostic method, the ROC plot allows deriving suitable thresholds.

In step d) of the methods of the present invention the amount of the cardiac troponin in the first sample and/or the amount in the second sample shall be compared to reference amounts.

Various reference amounts may apply.

For ruling out myocardial infarction (as set forth in (A)) the following reference amounts shall be used:
- 25 ng/l with respect to the second sample if the subject is 75 years old or older
- 12 ng/l with respect to the first sample

Accordingly, a preferred reference amount with respect to the second sample is 25 ng/l, if the subject is 75 years old or older. An amount of a cardiac troponin, in particular of troponin T of less than 25 ng/l, preferably, indicates that said suffer does not suffer from myocardial infarction, i.e. that myocardial infarction can be ruled out. Moreover, a further reference amount with respect to the first sample is 12 ng/l, if the subject is younger than 75 years. If the amount of the cardiac Troponin in the first sample is less than 12 ng/l, and if the difference between the amount of the cardiac troponin in the second sample and the amount in first sample is less than 3 ng/l, myocardial infarction can be ruled out.

For ruling in myocardial infarction (as set forth in (B)) the following reference amounts shall be used (for the specific algorithm see below):
- 12 ng/l with respect to the first sample
- 60 ng/l with respect to the first sample

For identifying a subject who requires further monitoring in order to diagnose myocardial infarction (as set forth in (C), "intermediate zone") the following reference amounts shall be used (for the specific algorithm see below):
- 12 ng/l with respect to the first sample
- 60 ng/l with respect to the first sample

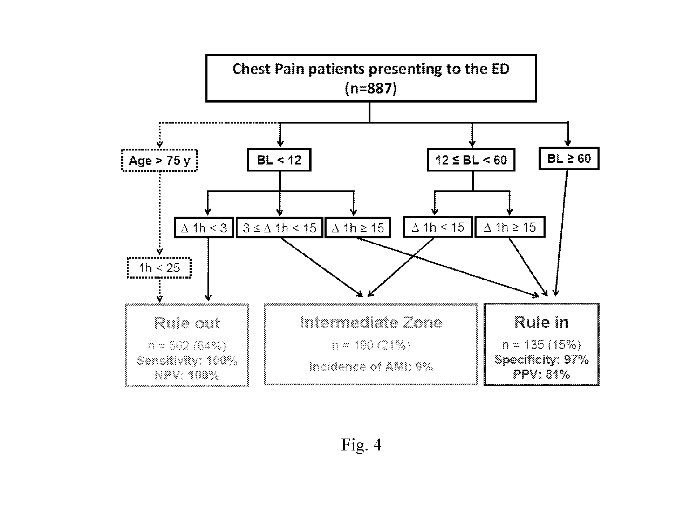

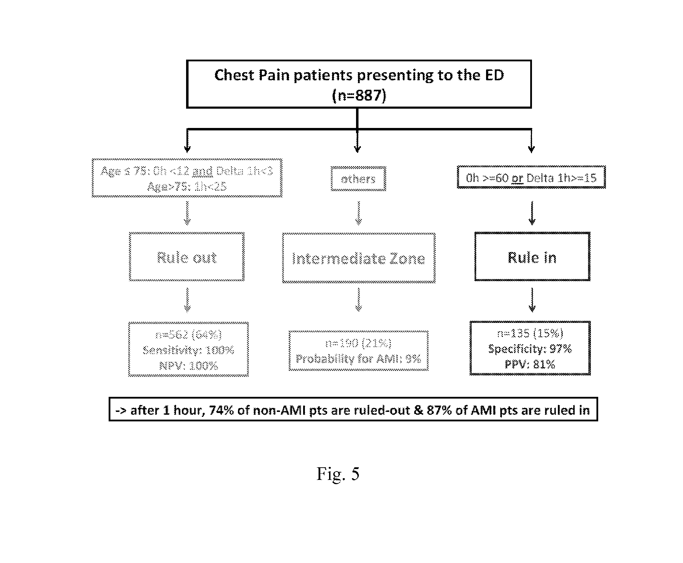

Preferred algorithms for diagnosing myocardial infarction are disclosed in Fig. 4 and 5, respectively. In particular, the following applies as diagnostic algorithm(s):

### (A) Rule out

Preferably, the subject does not suffer from myocardial infarction,
(i) if the subject is 75 years old or older, and if the amount of the cardiac troponin in the second sample is less than 25 ng/l, and/or
(ii) if the subject is younger than 75 years and if the amount of the cardiac Troponin in the first sample is less than 12 ng/l, and if the difference between the amount of the cardiac troponin in the second sample and the amount in first sample is less than 3 ng/l (and, thus, if the increase/decrease of the amount of a cardiac troponin in the second sample as compared to the amount of said cardiac troponin in said first sample is less than 3 ng/l)

Accordingly, myocardial infarction can be ruled out (and, thus, the diagnosis that the subject does not suffer from myocardial infarction can be made) if one or two of the aforementioned criteria are fulfilled (i.e. (i) or (ii), or (i) and (ii).

### (B) Rule in

Preferably, the subject suffers from myocardial infarction,
(i) if the amount of the cardiac Troponin in the first sample is less than 12 ng/l and if the difference between the amount of the cardiac Troponin in the second sample and the amount in the first sample is at least 15 ng/l (and, thus, if the increase or decrease the of the amount of the cardiac troponin in the second sample as compared to the amount of said cardiac troponin in said first sample is at least 15 ng/l),
(ii) if the amount of the cardiac troponin in the first sample is between 12 ng/l and less than 60 ng/l (and, thus, in particular larger than or equal to 12 ng/l and less than 60 ng/l) and if the difference between amount of the cardiac troponin in the second sample and the amount of the cardiac troponin in the first sample is at least 15 ng/l (and, thus, if the increase or decrease of the amount of the cardiac troponin in the second sample as compared to the amount of said cardiac troponin in said first sample is at least 15 ng/l), or
(iii) if the amount of the cardiac Troponin in the first sample is at least 60 ng/l.

Accordingly, myocardial infarction can be ruled in (and, thus, the diagnosis that the subject suffers from myocardial infarction can be made) if one of the aforementioned criteria is fulfilled (i.e. (i), (11), or (iii)).

### (C) Intermediate zone (further monitoring)

Preferably, the subject requires further monitoring, if (C) neither the criteria for (A), i.e. for ruling out MI, nor for (B), i.e. for ruling in MI, are fulfilled.

Accordingly, the subject, preferably, requires further monitoring in order to allow for diagnosing myocardial infarction,
(i) if the amount of the cardiac troponin in the first sample is less than 12 ng/l, and if the difference between the amount of the cardiac troponin in the second sample and the amount of the cardiac troponin in the first sample is larger than or equal to 3 ng/l but less than 15 ng/l (and, thus, if the increase or decrease of the amount of a cardiac troponin in the second sample as compared to the amount of said cardiac troponin in said first sample larger than or equal to 3 ng/l, but less than 15 ng/l), or
(ii) if the amount of the cardiac troponin in the first sample is larger than or equal to 12 and less than 60 ng/l and if the difference between the amount of the cardiac troponin in the second sample and the amount of the cardiac troponin in the first sample is less than 15 ng/l (and, thus, if the increase or decrease of the amount of a cardiac troponin in the second sample as compared to the amount of said cardiac troponin in said first sample is less than 15 ng/l).

If the subject requires further monitoring in order to diagnose MI, and thus in order to rule in or rule out myocardial infarction, the diagnosis of MI is made later. How to carry out a later diagnosis of myocardial infarction is well known in the art. For the diagnosis, the amount of a cardiac Troponin may be, preferably, determined in at least one further sample from said subject (and compared to a reference amount). Preferably, said at least one further sample, in particular a third sample, is obtained at least 2 hours, or more preferably, at least 3 hours, or even more preferably, at least four hours or, even more preferably, at least 6 hours after the second sample. It is further envisaged that the at least one further sample is obtained within 6 to 9 hours after presentation. The diagnosis of mycocardial infarction based on said at least one further sample can be carried out by the skilled person with out further ado, see Thygesen K, Alpert JS, White HD. Universal definition of myocardial infarction. Eur Heart J 2007;28:2525-38 which herewith is incorporated by reference with respect to its entire disclosure content.

Preferably, the subject suffers from MI, if the amount of said cardiac Troponin is larger than the 99th percentile in said at least one further samples, in one further sample is obtained within 6 to 9 hours after presentation. In all other cases, the subject, preferably, does not suffer from MI. The expressing "99^{th} percentile" (in particular of healthy population) in connection with myocardial infarction is well known in the art, see e.g. Thygesen K, Alpert JS, White HD. Universal definition of myocardial infarction. Eur Heart J 2007;28:2525-38 which herewith is incorporated by reference with respect to its entire disclosure content.

The definitions and explanations given herein apply mutatis mutandis to the following.

Moreover, the present invention relates to a method for ruling out myocardial infarction in a subject presenting with chest pain. Said method comprises the following steps:
a) determining the amount of a cardiac troponin in a first sample from the subject obtained at presentation to a physician, and
b) determining the amount of a cardiac troponin in a second sample from said subject obtained within one hour after the first sample,
wherein myocardial infarction can be ruled out,
(i) if the subject is 75 years old or older, and if the amount of the cardiac troponin in the second sample is less than 25 ng/l, and/or
(ii) if the subject is younger than 75 years and if the amount of the cardiac Troponin in the first sample is less than 12 ng/l, and if the difference between the amount of the cardiac troponin in the second sample and the amount in first sample is less than 3 ng/l (and, thus, if the increase/decrease of the amount of a cardiac troponin in the second sample as compared to the amount of said cardiac troponin in said first sample is less than 3 ng/l).

In a preferred embodiment of the aforementioned method, the method comprises the further steps:
c) assessing the difference between the amount of the cardiac troponin in the second sample and the amount of said cardiac troponin the first sample, and/or
d) comparing the amount of the cardiac troponin in the first sample and in the second sample to reference amounts.

Moreover, the present invention relates to a method for ruling in myocardial infarction in a subject presenting with chest pain. Said method comprises the following steps:
a) determining the amount of a cardiac troponin in a first sample from the subject obtained at presentation to a physician, and
b) determining the amount of a cardiac troponin in a second sample from said subject obtained within one hour after the first sample,
wherein myocardial infarction can be ruled in,
(i) if the amount of the cardiac Troponin in the first sample is less than 12 ng/l and if the difference (and thus, the decrease or increase) between the amount of the cardiac Troponin in the second sample and the amount in the first sample is at least 15 ng/l,
(ii) if the amount of the cardiac troponin in the first sample is between 12 and less than 60 ng/l (and, thus, is in particular larger than or equal to 12 ng/l and less than 60 ng/l) and if the difference (and, thus, the increase or decrease) between amount of the cardiac troponin in the second sample and the amount of the cardiac troponin in the first sample is at least 15 ng/l, or
(iii) if the amount of the cardiac Troponin in the first sample is at least 60 ng/l.

In a preferred embodiment of the aforementioned method, the method comprises the further steps:
c) assessing the difference between the amount of the cardiac troponin in the second sample and the amount of said cardiac troponin the first sample, and/or
d) comparing the amount of the cardiac troponin in the first sample to reference amounts,

In a preferred embodiment of the methods of the present invention, said methods further comprises the step of recommending a therapeutic measure if a myocardial infarction has been diagnosed. Accordingly, by carrying out the method of the present invention, a subject can be identified who is susceptible to a certain therapeutic measure as described herein below.

The term "recommending" as used herein means establishing a proposal for a therapy which could be applied to the subject. However, it is to be understood that applying the actual therapy whatsoever is not comprised by the term. The therapy to be recommended depends on the outcome of the diagnosis provided by the method of the present invention. The recommendation step referred to above can also, preferably, be automated. Preferably, the diagnosis or aid for diagnosis obtained from the step b) of the method of the present invention, i. e. the diagnostic result of the method, will be used to search a database comprising recommendations of therapeutic measures for the individual possible diagnostic results.

Preferred therapeutic measures for subjects suffering from MI are well known in the art and, e.g., described in Kushner FG et al. 2009 Focused Updates: ACC/AHA Guidelines for the Management of Patients With ST-Elevation Myocardial Infarction (updating the 2004 Guideline and 2007 Focused Update) and ACC/AHA/SCAI Guidelines on Percutaneous Coronary Intervention (updating the 2005 Guideline and 2007 Focused Update): a report of the American College of Cardiology Foundation/American Heart Association Task Force on Practice Guidelines. Circulation. 2009 Dec 1;120(22):2271-306, or in Bassand JP, Hamm CW, Ardissino D, Boersma E, Budaj A, Fernandez-Aviles F, et al. Guidelines for the diagnosis and treatment of non-ST-segment elevation acute coronary syndromes. Eur Heart J 2007;28:1598-660. Preferably, said therapeutic measure is a cardiac intervention. The term "cardiac intervention", preferably, encompasses those treatment regimens which comprise an intervention by surgery, microsurgery or other invasive therapies affecting the cardiovascular system and, preferably, the heart. Preferably, cardiac interventions as used herein are treatment regimens which aim to restore the proper oxygen supply of the heart. This is, preferably, achieved by restoring the blood flow throughout the blood vessels supporting the heart, i.e. the coronary blood vessels. Those blood vessels may be impaired due to, e.g., thrombotic or atherosclerotic plaques. Accordingly, cardiac interventions shall, preferably, comprise a destruction and/or removal of such plaques and a restoration of the vessel, if necessary. Preferred cardiac interventions in accordance with the present invention are selected from the group consisting of percutaneous coronary angioplasty, percutaneous transluminal coronary balloon angioplasty, laser angioplasty, coronary stent implantation, bypass implantation or intraluminal techniques aiming to restore blood flow, vessel patency, stabilize plaque, and/or reduce intracoronary thrombus load.

Accordingly, the present invention relates to a method of identifying a subject being in need for cardiac intervention, comprising the steps of:
a) determining the amount of a cardiac troponin in a first sample from the subject obtained at presentation to a physician, and
b) determining the amount of a cardiac troponin in a second sample from said subject obtained within one hour after the first sample,

(A) wherein the subject is not in need for cardiac intervention,
   (i) if the subject is older than 75 years and the amount of the cardiac troponin in the second sample is less than 25 ng/l, and/or
   (ii) if the subject is younger than 75 years and if the amount of the cardiac Troponin in the first sample is less than 12 ng/l, and if the difference (and thus, the decrease or increase) between the amount of the cardiac troponin in the second sample and the amount in first sample is less than 3 ng/l,
(B) wherein the subject is in need for cardiac intervention,
   (i) if the amount of the cardiac Troponin in the first sample is less than 12ng/l and if the difference (and, thus, the decrease or increase) between the amount of the cardiac Troponin in the second sample and the first sample is at least 15 ng/l,
   (ii) if the amount of the cardiac troponin in the first sample is between 12 and less than 60 ng/l (and, thus, is in particular larger than or equal to 12 ng/l and less than 60 ng/l) and if the difference (and, thus, the decrease or increase) between amount of the cardiac troponin in the second sample and the amount of the cardiac troponin in the first sample is at least 15 ng/l, or
   (iii) if the amount of the cardiac Troponin in the first sample is at least 60 ng/l.

In a preferred embodiment the method, preferably, comprises the further steps:
c) assessing the difference between the amount of the cardiac troponin in the second sample and the amount of said cardiac troponin the first sample, and/or
d) comparing the amount of the cardiac troponin in the first sample and the amount in the second sample to reference amounts,

Preferably, the subject requires further monitoring, if (C) neither the criteria (A) nor (B) are fulfilled.

Accordingly, the subject, preferably, requires further monitoring in order to identify a subject being in need of cardiac intervention,
(i) if the amount of the cardiac troponin in the first sample is less than 12 ng/l, and if the difference between the amount of the cardiac troponin in the second sample and the amount of the cardiac troponin in the first sample is larger than or equal to 3 ng/l but less than 15 ng/l (and, thus, if the increase or decrease of the amount of a cardiac troponin in the second sample as compared to the amount of said cardiac troponin in said first sample larger than or equal to 3 ng/l, but less than 15 ng/l), or
(ii) if the amount of the cardiac troponin in the first sample is larger than or equal to 12 ng/l and less than 60 ng/l and if the difference between the amount of the cardiac troponin in the second sample and the amount of the cardiac troponin in the first sample is less than 15 ng/l (and, thus, if the increase or decrease of the amount of a cardiac troponin in the second sample as compared to the amount of said cardiac troponin in said first sample is less than 15 ng/l).

The phrase "a subject in need of cardiac intervention" as used herein relates to a subject who, preferably, suffers from myocardial infarction (as diagnosed by method described above). It will be understood that further therapy is at least beneficial for such subject.

Moreover, by carrying out the present invention, patients can be identified which shall be subjected to coronary angiography in order to assess the degree of occlusion of the coronary arteries. Preferably, a subject shall be subjected to coronary angiography if the subject suffers from MI (and, thus, if the diagnosis MI has been made by carrying the method of the present invention).

Accordingly, the present invention relates to a method of identifying a subject being in need for coronary angiography comprising the steps of:
a) determining the amount of a cardiac troponin in a first sample from the subject obtained at presentation to a physician, and
b) determining the amount of a cardiac troponin in a second sample from said subject obtained within one hour after the first sample,

(A) wherein the subject is not in need for coronary angiography,
   (i) if the subject is older than 75 years and the amount of the cardiac troponin in the second sample is less than 25 ng/l, and/or
   (ii) if the subject is younger than 75 years and if the amount of the cardiac Troponin in the first sample is less than 12 ng/l, and if the difference (and thus, the decrease or increase) between the amount of the cardiac troponin in the second sample and the amount in first sample is less than 3 ng/l,
(B) wherein the subject is in need for coronary angiography,
   (i) if the amount of the cardiac Troponin in the first sample is less than 12ng/l and if the difference (and, thus, the decrease or increase) between the amount of the cardiac Troponin in the second sample and the first sample is at least 15 ng/l,
   (ii) if the amount of the cardiac troponin in the first sample is between 12 and less than 60 ng/l (and, thus, is in particular larger than or equal to 12 ng/l and less than 60 ng/l) and if the difference (and, thus, the decrease or increase) between amount of the cardiac troponin in the second sample and the amount of the cardiac troponin in the first sample is at least 15 ng/l, or
   (iii) if the amount of the cardiac Troponin in the first sample is at least 60 ng/l.

In a preferred embodiment the method, preferably, comprises the further steps:
c) assessing the difference between the amount of the cardiac troponin in the second sample and the amount of said cardiac troponin the first sample, and/or
d) comparing the amount of the cardiac troponin in the first sample and the amount in the second sample to reference amounts,

Preferably, the subject requires further monitoring, if (C) neither the criteria (A) nor (B) are fulfilled.

Moreover, the present invention relates to the in vitro use of a cardiac Troponin or of a detection agent which specifically binds to a cardiac troponin in a first and second sample from a subject presenting with chest pain for i) diagnosing myocardial infarction in said subject, ii) for ruling in myocardial infarction in said subject, or iii) for ruling out myocardial infarction in said subject, wherein the first sample has been obtained at presentation and wherein the second sample has been obtained within one hour after said first sample.

Preferred diagnostic algorithms for i) diagnosing myocardial infarction, for ii) ruling in myocardial infarction, or for iii) ruling out myocardial infarction have been described elsewhere herein.

Preferably, the diagnostic algorithm for diagnosing myocardial infarction comprises the algorithms as set forth under (A), (B), and, optionally, (C).

Preferably, the diagnostic algorithm for ruling-out myocardial infarction is the algorithm as set forth under (A).

Preferably, the diagnostic algorithm for ruling-in myocardial infarction is the algorithm as set forth under (B).

The term "detection agent" as used herein refers to an agent which is capable of specifically recognizing and binding to one of the biomarker, i.e. of the cardiac Troponin, referred to above when present in a sample. Moreover, said agent shall allow for direct or indirect detection of the complex formed by the said agent and the biomarker. Direct detection can be achieved by including into the agent a detectable label. Indirect labelling may be achieved by a further agent which specifically binds to the complex comprising the biomarker and the detection agent wherein the said further agent is than capable of generating a detectable signal. Suitable compounds which can be used as detection agents are well known in the art. Preferably, the detection agent is an antibody or which specifically binds to the cardiac troponin. Preferred antibodies for the determination of a cardiac Troponin, in particular for a Troponin T have been described elsewhere herein. Antibodies as referred to herein include both polyclonal and monoclonal antibodies, as well as fragments thereof, such as Fv, Fab and F(ab)2 fragments that are capable of binding antigen or hapten. In a preferred embodiment, the detection agent for Troponin T comprises two monoclonal antibodies which specifically bind to epitopes located in the central region of the Troponin T polypeptide, in particular to amino acid positions 125-131 and 135-147, respectively. These antibodies are, e.g. described by Giannitsis et al. (loc. cit.).

In a preferred embodiment of the present inventions the cardiac troponin detecting means or assay has a lower detection limit of cardiac troponin of up to 3 ng/l. The lower detection limit, also referred to as the limit of blank (LoB), Limit of Detection (LoD) or Limit of Quantitation (LoQ). LoB may be determined in accordance with the CLSI (Clinical and Laboratory Standards Institute) EP17-A requirements (for more details see e.g. Tholen, K. et al., Protocols for determination of limits of detection and limits of quantitation; approved guideline Vol. 24, n° 34, NCCLS, Wayne, PA, USA (2004) NCCLS publication EP17-A.).

Preferably, the amount of the cardiac Troponin (in particular of Troponin T) is determined with the aforementioned assays, in particular with the assay described by Giannitsis et al. (loc.cit.). Preferably, the reference amounts and differences between the amount of the cardiac Troponin in the first and in the second sample as set forth in algorithms (A), (B) and (C) are based on the use on the assay that was used in the Examples, in particular on the use of the Roche hs cTnT as described by Giannitsis et al. (loc.cit.). The person skilled in the art knows that the amounts of the cardiac Troponin may slightly differ based on the assay used for the determination of the amount of the cardiac Troponin. However, suitable reference amounts and differences between the amount of the cardiac Troponin in the first and in the second sample which reference amounts and differences correspond to the reference amounts and differences set forth herein can be determined by the skilled person without further ado, see e.g. Reichlin et al, N Engl J Med 2009;361:858-67. In particular, the reference amounts and the differences (between the amount of the cardiac troponin in the first sample and the second sample being indicative for ruling-in or ruling out MI) for other assays can be determined by carrying out the analysis as described in the Examples.

The present invention relates to a device for diagnosing whether a subject who presents with chest pain suffers from myocardial infarction, or not, said device comprising:
a) an analyzing unit comprising a detection agent for a cardiac Troponin which allows for the determination of the amount of a cardiac Troponin in a first and second sample of a subject; and
b) an evaluation unit comprising a data processor, wherein said data processor has implemented an algorithm for diagnosing myocardial infarction, and wherein the algorithm is an algorithm as defined above in connection with the method of the invention (i.e. in connection with the method for diagnosing myocardial infarction).

Preferably, the algorithm includes the algorithm as set forth under (A) and (B), and, optionally, (C).

Preferably, said data processor is capable of comparing the amount of the cardiac troponin in the first sample and the amount of the cardiac troponin in the second sample to reference amounts, and is further capable of assessing the difference between the amount of the cardiac troponin in the second sample and the amount of said cardiac troponin the first sample.

The present invention relates to a device for ruling out myocardial infarction in a subject who presents with chest pain suffers, said device comprising:
a) an analyzing unit comprising a detection agent for a cardiac Troponin which allows for the determination of the amount of a cardiac Troponin in a first and second sample of a subject; and
b) an evaluation unit comprising a data processor, wherein said data processor has implemented an algorithm for ruling out, and wherein the algorithm is an algorithm as defined above in connection with the method of the invention (i.e. in connection with the method for ruling out myocardial infarction).

Preferably, said data processor is further capable of comparing the amount of the cardiac troponin in the first sample and the amount of the cardiac troponin in the second sample to reference amounts, and capable of assessing the difference between the amount of the cardiac troponin in the second sample and the amount of said cardiac troponin the first sample,

Preferably, the algorithm includes the algorithm as set forth under (A).

The present invention relates to a device for ruling in myocardial infarction in a subject who presents with chest pain suffers, said device comprising:
a) an analyzing unit comprising a detection agent for a cardiac Troponin which allows for the determination of the amount of a cardiac Troponin in a first and second sample of a subject; and
b) an evaluation unit comprising a data processor, wherein said data processor has implemented an algorithm for ruling in, and wherein the algorithm is an algorithm as defined above in connection with the method of the invention (i.e. in connection with the method for ruling in myocardial infarction).

Preferably, the algorithm includes the algorithm as set forth under (B).

Preferably, said data processor is further capable of comparing the amount of the cardiac troponin in the first sample and the amount of the cardiac troponin in the second sample to reference amounts, and capable of assessing the difference between the amount of the cardiac troponin in the second sample and the amount of said cardiac troponin the first sample,

The term "device" as used herein relates to a system comprising the aforementioned units operatively linked to each other as to allow the diagnosis according to the methods of the invention. Preferred detection agents which can be used for the analyzing unit are disclosed elsewhere herein. The analyzing unit, preferably, comprises said detection agents in immobilized form on a solid support which is to be contacted to the sample comprising the biomarkers the amount of which is to be determined. Moreover, the analyzing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references can be derived from samples of subjects to be used for the generation of reference amounts as described elsewhere herein above. The diagnostic results may be given as output of parametric diagnostic raw data, preferably, as absolute or relative amounts. It is to be understood that these data may need interpretation by the clinician. However, also envisage are expert system devices wherein the output comprises processed diagnostic raw data the interpretation of which does not require a specialized clinician. Preferably, the device of the present invention can be used to carry out the aforementioned method of the present invention in an automated manner.

The figures show:

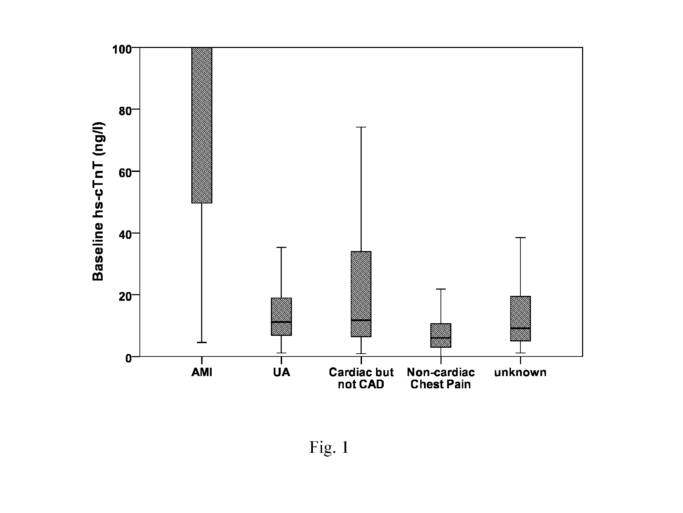

**Figure 1****:** Baseline hs-cTnT levels at presentation to the emergency department in all patients according to the adjudicated final diagnoses. Boxes represent IQR's (interquartile ranges), while whiskers display ranges (without outliers further than 1.5 IQR's from the end of the box).

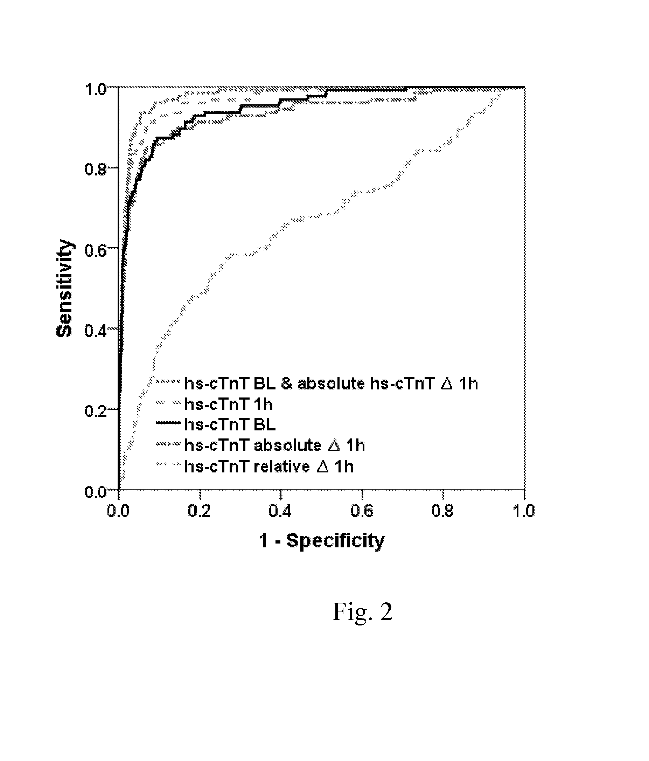

**Figure 2****:** Receiver operating characteristics curves displaying the diagnostic accuracy in the diagnosis of AMI of hs-cTnT (high sensitive Troponin T) values at baseline (BL) and 1h as well as of absolute and relative changes within the first hour (Δ 1h) and of the combination of BL with absolute 1h changes.

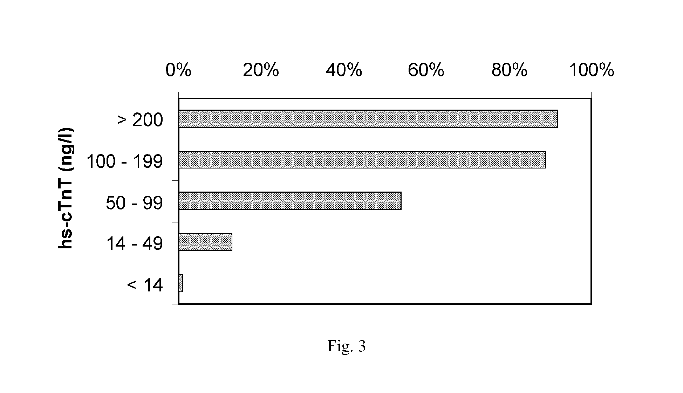

**Figure 3****:** Incidence of AMI according to absolute levels of hs-cTnT (ng/l) at presentation to the emergency department in all patients.
**Figure 4****:** Algorithm for diagnosis of AMI using hs-cTnT in patients presenting with chest pain whereby hs-cTnT values are presented in ng/l. Abbreviations are as follows: ED = emergency department; BL = baseline levels of hs-cTnT; Δ 1h = absolute changes of hs-cTnT within first hour; NPV = negative predicitve value; PPV = positive predictive value.
**Figure 5****:** Alternative presentation of the algorithm for diagnosis of AMI using hs-cTnT in patients presenting with chest pain whereby hs-cTnT values are presented in ng/l. Abbreviations are as follows: ED = emergency department; BL = baseline levels of hs-cTnT; Δ 1h = absolute changes of hs-cTnT within first hour; NPV = negative predictive value; PPV = positive predictive value.

All references cited in this specification are herewith incorporated by reference with respect to their entire disclosure content and the disclosure content specifically mentioned in this specification.

### Examples:

### Example 1: Methods

### Study design and population

Advantageous Predictors of Acute Coronary Syndrome Evaluation (APACE) is an ongoing prospective international multicenter study designed and coordinated by the University Hospital Basel Reichlin T, Hochholzer W, Bassetti S, et al. Early diagnosis of myocardial infarction with sensitive cardiac troponin assays. N Engl J Med 2009;361:858-67, Reichlin T, Hochholzer W, Stelzig C, et al. Incremental value of copeptin for rapid rule out of acute myocardial infarction. J Am Coll Cardiol2009;54:60-8). From April 2006 to June 2009, a total of 1247 consecutive patients presenting to the emergency department (ED) with acute chest pain symptoms suggestive of AMI such as acute chest pain and angina pectoris with an onset or peak within the last 12 hours were recruited. Patients with terminal kidney failure requiring dialysis were excluded. The study was carried out according to the principles of the Declaration of Helsinki and approved by the local ethics committees. Written informed consent was obtained from all patients. The authors designed the study, gathered and analyzed the data, vouch for the data and analysis, wrote the paper, and decided to publish. There were no agreements concerning confidentiality between the sponsors and authors or institutions.

### Routine clinical assessment

All patients underwent an initial clinical assessment that included clinical history, physical examination, 12-lead ECG, continuous ECG-monitoring, pulse oximetry, standard blood tests and chest radiography. CTnI or cTnT, CK-MB and myoglobin were measured at presentation and after 6-9 hours or as long as clinically indicated. Timing and treatment of patients were left at discretion of the attending physician.

### Adjudicated final diagnosis

To determine the final diagnosis for each patient, two independent cardiologists reviewed all available medical records (including patient history, physical examination, results of laboratory testing including local cTn values, radiologic testing, ECG, echocardiography, cardiac exercise test, coronary angiography) pertaining to the patient from the time of ED presentation to 60-day follow-up. In situations of diagnostic disagreement, cases were reviewed and adjudicated in conjunction with a third cardiologist.

AMI was defined as recommended in current guidelines. In brief, AMI was diagnosed when there was evidence of myocardial necrosis in a clinical setting consistent with myocardial ischemia. Necrosis was diagnosed by at least one value of the cTn above the 99^{th} percentile with an imprecision of less than 10%. A significant rise and/or fall was defined as a change of at least 30% of the 99^{th} percentile or the 10% CV level respectively within 6 to 9 hours after the first sample has been obtained (Apple FS, Jesse RL, Newby LK, Wu AH, Christenson RH., National Academy of Clinical-Biochemistry and IFCC Committee for Standardization of Markers of CardiacDamage Laboratory Medicine Practice, Guidelines: Analytical issues forbiochemical markers of acute coronary syndromes. Circulation 2007;115:e352-5; and Apple FS Wu AH, Jaffe AS. European Society of Cardiologyand American College of Cardiology guidelines for redefinition of myocardial infarction: how to useexisting assays clinically and for clinical trials. Am Heart J 2002; 144:981-6.

The following cTn assays were used for the adjudication of the final diagnosis onsite: Abbott Axsym cTnI ADV, Beckmann Coulter Accu cTnI, and Roche cTnT fourth generation. All three are well-validated current standard cTn assays with comparable performance in the diagnosis of AMI (see Apple et al., loc. Cit). UA was diagnosed in patients with normal cTn levels and typical angina at rest, a deterioration of a previously stable angina, in cases of positive cardiac exercise testing or cardiac catheterization with coronary arteries found to have a stenosis of 70% or greater, and in ambiguous cases in which follow-up information revealed AMI or a sudden, unexpected cardiac death within 60 days. Further predefined diagnostic categories included cardiac but not coronary symptoms (e.g. perimyocarditis, tachyarrhythmias), NCCP, and symptoms of unknown origin. If AMI was excluded in the ED, but no sufficient further diagnostic procedures were performed for conclusive diagnosis, symptoms were classified as to be of unknown origin.

### Investigational hs-cTnT analysis

Blood samples for determination of hs-cTnT (Roche Diagnostics) were collected in serum tubes at presentation to the ED. Additional samples were collected after 1 hour (+/-15 min). Further samples were obtained after 2, 3, and 6 hours. Serial sampling was discontinued when the diagnosis of AMI was certain and treatment required transferring the patient to the catheter laboratory or coronary care unit. Of the 1247 patients, samples at presentation as well as after 1h were available for measurement of hs-cTnT in 887 patients. After centrifugation, samples were frozen at -80°C until assayed in a blinded fashion using the Elecsys 2010 (Roche Diagnostics) in a dedicated core laboratory. For hs-cTnT, limit of blank (LoB) and limit of detection (LoD) have been determined to be 3 ng/l and 5 ng/l, an imprecision corresponding to 10% coefficient of variation (CV) was reported at 13 ng/L and the 99^{th}-percentile of a healthy reference population at 14 ng/L (GiannitsisE, Kurz K, Hallermayer K, Jarausch J, Jaffe AS, Katus HA. Analytical Validation of a High-Sensitivity Cardiac Troponin T Assay. Clin Chem 2009).

Troponin T was determined using Roche's electrochemiluminescence ELISA sandwich test Elecsys Troponin T hs (high sensitive) (STAT (Short Turn Around Time)) 18 min assay as described by Giannitsis et al. The test employs two monoclonal antibodies specifically directed against human cardiac troponin T. The antibodies recognize two epitopes (amino acid position 125-131 and 136-147) located in the central part of the cardiac troponin T protein, which consists of 288 amino acids. The hs-TnT assay allows a measurement of troponin T levels in the range of 3 to 10000 pg/mL (without dilution of the sample).

Glomerular filtration rate was calculated using the abbreviated Modification of Diet in Renal Disease formula (Levey AS, Coresh J, Greene T, et al. Using standardized serum creatinine values in the modification of diet in renal disease study equation for estimating glomerular filtration rate. Ann Intern Med 2006;145:247-54).

### Statistical analysis

Continuous variables are presented as means ± SD or medians with interquartile range (IQR), categorical variables as numbers and percentages. Continuous variables were compared with the Mann-Whitney U test and categorical variables using the Pearson chi-square test. Receiver operating characteristic (ROC) curves were constructed to assess prognostic accuracy of levels and changes of hs-cTnT to predict death or AMI during follow-up in UA patients. Comparison of areas under the ROC curves was performed as recommended by DeLong (DeLongER, DeLong DM, Clarke-Pearson DL. Comparing the areas under two or more correlated receiver operating characteristic curves: a non-parametric approach.Biometrics 1988;44:837-45).

The algorithm for use of hs-cTnT was developed in a derivation sample of 444 patients selected by stratified randomization according to "presence vs. absence of AMI" and "age ≥75 years vs. < 75 years". Optimal thresholds for rule-in were obtained by minimizing the sum of false positive and false negative decisions. Optimal thresholds for rule-out were selected to allow for a 100% sensitivity and negative predictive value (NPV). The algorithm was then tested prospectively in a validation data set of the remaining 443 subjects. The rule-in criterion developed as described above was further validated using 1000 bootstrap samples and the bootstrap 95% confidence intervals (95% CI) were calculated. Contrariwise, we estimated the probability of an AMI patient to be falsely ruled-out in a virtual infinite patient sample according to the normal distribution of Box-Cox transformed hs-cTnT baseline values and 1 hour absolute changes.

All hypothesis testing was two-tailed and a p-value of less than 0.05 was considered statistically significant. All statistical analyses were performed using SPSS for Windows 19.0 (SPSS Inc, Chicago, IL), MedCalc 9.6.4.0 (MedCalc Software) and SAS 9.2 (SAS Institute).

### Example 2: Results

### Characteristics of patients

Among the 887 patients presenting to the ED with acute chest pain, the adjudicated final diagnosis was AMI in 127 patients (14%), unstable angina (UA) in 125 (14%), cardiac symptoms of origin other than coronary artery disease in 124 (14%), non-cardiac symptoms in 437 (49%), and symptoms of unknown origin in 74 (8%). Of the AMI patients, 88% had non-ST-elevation myocardial infarction (NSTEMI) and 12% had ST-elevation myocardial infarction (STEMI). Baseline characteristics of the patients are shown in Table 1.

### Diagnostic information provided by hs-cTnT baseline levels

Baseline levels of hs-cTnT were significantly higher in patients with AMI as compared to the other final diagnoses (Figure 1). Baseline values were similar in men and women, (median in men 9.1 ng/l, 95% CI 4.6 - 21.8 ng/l vs. median in women 8.5 ng/l, 95% CI 3.8 - 20.6 ng/l, p=0.40), but significantly higher in patients 75 years of age or older compared to those younger than 75 years ((median in ≥ 75 years 20.2 ng/l, 95% CI 11.5 - 51.7 ng/l vs. median in < 75 years 6.95 ng/l, 95% CI 3.3 - 13.7 ng/l, p=0.02).

The diagnostic accuracy of hs-cTnT baseline levels for AMI as quantified by the AUC was very high and reached 0.95 (95% confidence interval [CI], 0.93-0.97, Figure 2). The incidence of AMI in patients presenting with acute chest pain differed significantly according to quantitative levels of hs-cTnT (Figure 3). In patients with hs-cTnT <14 ng/l (99^{th} percentile of healthy individuals), the incidence of AMI was 1.4% and there was a rise to 13% in patients with 14-49 ng/l, 54% in patients with 50 - 99 ng/l, 89% in patients with 100 -199 ng/l and finally 92% in patients with levels ≥ 200 ng/l (p=0.66 for comparisons of 100-199 ng/l vs. ≥ 200 ng/l, p<0.001 for all other comparisons). Of all patients, 36% had hs-cTnT baseline levels above the 99^{th} percentile of healthy individuals (14 ng/l). Using this value as a qualitative cut-off for baseline levels to diagnose AMI resulted in a sensitivity of 94%, a negative predictive value of 99%, a specificity of 74% and a positive predictive value of 38%.

### Diagnostic information provided by hs-cTnT changes within the first hour

The diagnostic accuracy of hs-cTnT levels obtained 1h after presentation was significantly higher compared to that of baseline values (AUC 0.97, 95% CI 0.95-0.98, p=0.007 for comparison, figure 2). Absolute values of absolute changes within the first hour showed a very high diagnostic accuracy as well (AUC 0.93, 95% CI 0.90-0.96) and were superior compared to relative changes (AUC 0.66, 95% CI 0.61-0.72, p=0.72 for comparison).

### Use of hs-cTnT for the diagnosis of AMI

Combining baseline levels of hs-cTnT with absolute changes within the first hour further improved the diagnostic accuracy of both baseline and 1h levels (AUC 0.98, 95% CI 0.97-0.99, p<0.001 and p=0.03 for comparison with baseline and 1h levels).

For use in clinical practice, an algorithm incorporating baseline hs-cTnT values as well as absolute changes within the first hour was developed in a derivation sample of 444 randomly selected patients. For rule-in of AMI, the optimal thresholds in the derivation sample after minimizing the sum of false positive and false negative decisions were 58.2 ng/l for baseline hs-cTnT and 17.5 ng/l for absolute changes within the first hour. Additional use of gender, ECG features or time since onset of symptoms could not further improve the accuracy of the algorithm. We then rounded the hs-cTnT thresholds to 60 ng/l for baseline and 15 ng/l for absolute 1 hour changes respectively, thereby slightly strengthening the baseline criterion while loosening the change criterion. For rule-out of AMI, the optimal thresholds to allow for a 100% sensitivity and NPV were 12 ng/l for baseline hs-cTnT and 3 ng/l for absolute changes within the first hour in patients < 75 years and a 1 hour hs-cTnT of 25 ng/l for patients ≥ 75 years.

The algorithm was then tested prospectively in a validation sample of the remaining 443 subjects. Baseline characteristics of the patients in the derivation and the validation sample were similar and are shown in Table 2. The performance indices of the final algorithm in the derivation cohort, the validation cohort and the overall cohort are shown in Table 3 and the final algorithm is depicted in Figure 4. It allowed for a rule-out of AMI in 74% of non-AMI patients within 1 hour with a sensitivity and negative predictive value of 100% in the overall cohort. On the other hand side, 87% of all AMI patients could be ruled-in within 1 hour with a specificity of 97% and a positive predictive value of 81%. The final adjudicated diagnoses in patients falsely ruled-in for AMI (n=25) based on the algorithm were cardiac arrhythmias (n=8), myocarditis (n=4), acute heart failure (n=4), unstable angina (n=2), pulmonary embolism (n=1), takotsubo cardiomyopathy (n=1) and chest pain of unknown origin (n=5).

Using 1000 bootstrap samples, the calculated 95% CI for the rule-in thresholds were 55 - 92 ng/l at baseline and 9 - 47 ng/l for the absolute changes within the first hour. This resulted in a 95% CI of 95 - 98% for specificity and 76 - 87% for PPV. Contrariwise, we estimated the probability of an AMI-patient in a virtual infinite patient sample to be falsely ruled-out according to the algorithm by analyzing Gaussian curves of Box-Cox transformed baseline levels of hs-cTnT and 1 hour absolute changes. In the subsample of AMI-patients under age 75, the simulation of the approximating joint normal distributions of the transformed variables for baseline (lambda=-0.1) and 1 hour absolute changes (lambda=-0.01) resulted in a probability 0.02 for an AMI-patient to be falsely ruled-out. In the subsample of AMI-patients ≥ 75 years, the model of Box-Cox transformed 1 hour hs-cTnT values (lambda=-0.19) resulted in an estimated probability of 0.017 for an AMI-patient to have a hs-cTnT 1 hour value of < 25 ng/l.

| Table 1 | | Baseline Characteristics of the Patients | | | | |
|---|---|---|---|---|---|---|
| | | | **All patients** | **Acute MI** | **Others** | **P Value** |
| | | | **(n=887)** | **(n=127)** | **(n=760)** | |
| Age - yr | | | 64 (51 - 75) | 74 (61 - 82) | 63 (50 - 74) | < 0.001 |
| Male gender - no. (%) | | | 599 (68) | 87 (69) | 512 (67) | 0.80 |
| Risk factors - no. (%) | | | | | | |
| | Hypertension | | 568 (64) | 93 (73) | 475 (63) | 0.02 |
| | Hypercholesterolemia | | 415 (47) | 62 (49) | 353 (46) | 0.62 |
| | Diabetes | | 180 (20) | 31 (24) | 149 (20) | 0.21 |
| | Current smoking | | 204 (23) | 31 (24) | 173 (23) | 0.68 |
| | History of smoking | | 323 (36) | 46 (36) | 277 (36) | 0.96 |
| History - no. (%) | | | | | | |
| | Coronary artery disease | | 324 (37) | 54 (43) | 270 (36) | 0.13 |
| | Previous myocardial infarction | | 222 (25) | 39 (31) | 183 (25) | 0.11 |
| | Previous revascularization | | 241 (27) | 33 (26) | 208 (27) | 0.75 |
| | Peripheral artery disease | | 59 (7) | 14 (11) | 45 (6) | 0.03 |
| | Previous stroke | | 54 (6) | 19 (15) | 35 (5) | < 0.001 |
| | Creatinine clearance - (ml/min/m2) | | 89 (71 - 106) | 76 (61 - 100) | 90 (73 - 107) | < 0.001 |
| ECG findings - no. (%)† | | | | | | |
| | Left bundle branch block | | 35 (4) | 13 (10) | 22 (3) | < 0.001 |
| | ST-segment elevation | | 25 (3) | 13 (10) | 12 (2) | < 0.001 |
| | ST-segment depression | | 91 (10) | 38 (30) | 53 (7) | < 0.001 |
| | T-wave inversion | | 63 (7) | 13 (10) | 50 (7) | 0.14 |
| | No significant ECG abnormalities | | 673 (76) | 50 (39) | 623 (82) | < 0.001 |

| | | | | | | |
|---|---|---|---|---|---|---|
| t ECG denotes electrocardiogram | | | | | | |

| **Table 2** | | **Baseline characteristics of the patients in the derivation and validation cohort** | | | |
|---|---|---|---|---|---|
| | | | **Derivation cohort** | **Validation cohort** | **P Value** |
| | | | **(n=444)** | **(n=443)** | |
| Age - yr | | | 65 (53 - 75) | 63 (50 - 75) | 0.13 |
| Male gender - no. (%) | | | 287 (65) | 312 (70) | 0.07 |
| Risk factors - no. (%) | | | | | |
| | Hypertension | | 300 (68) | 268 (61) | 0.03 |
| | Hypercholesterolemia | | 208 (47) | 207 (47) | 0.97 |
| | Diabetes | | 91(21) | 89 (20) | 0.88 |
| | Current smoking | | 108 (24) | 96 (22) | 0.35 |
| | History of smoking | | 151 (34) | 172 (39) | 0.14 |
| History - no. (%) | | | | | |
| | Coronary artery disease | | 165 (37) | 19 (36) | 0.69 |
| | Previous myocardial infarction | | 117 (26) | 105(24) | 0.36 |
| | Previous revascularization | | 125 (28) | 116 (26) | 0.51 |
| | Peripheral artery disease | | 26 (6) | 33 (7) | 0.34 |
| | Previous stroke | | 28 (6) | 26 (6) | 0.79 |
| | Creatinine clearance - (ml/min/m2) | | 89 (69 - 106) | 89 (71 - 107) | 0.56 |

| Table 3 | | Performance of the hs-cTnT algorithm for rule-in and rule-out of AMI | | | |
|---|---|---|---|---|---|
| | | | **Overall cohort** | **Derivation cohort** | **Validation cohort** |
| | | | **(n=887)** | **(n=444)** | **(n=443)** |
| Nr. of patients diagnosed after 1 hour | | | 697 (79) | 346 (78) | 351 (79) |
| Rule-out | | | | | |
| | Sensitivity | | 100% | 100% | 100% |
| | Negative Predictive value | | 100% | 100% | 100% |
| Rule-in | | | | | |
| | Specificity | | 97% | 95% | 98% |
| | Positive Predicitve value | | 81% | 76% | 88% |

## Claims

1. A method for diagnosing myocardial infarction in a subject presenting with chest pain, comprising the steps of:
a) determining the amount of a cardiac troponin in a first sample from the subject obtained at presentation to a physician, and
b) determining the amount of a cardiac troponin in a second sample from said subject obtained within one hour after the first sample,
(A) wherein the subject does not suffer from myocardial infarction,
(i) if the subj ect is 75 years old or older and the amount of the cardiac troponin in the second sample is less than 25 ng/l, or
(ii) if the subject is younger than 75 years and the amount of the cardiac Troponin in the first sample is less than 12 ng/l, and if the difference between the amount of the cardiac troponin in the second sample and the amount in first sample is less than 3 ng/l,
(B) wherein the subject suffers from myocardial infarction,
(i) if the amount of the cardiac Troponin in the first sample is less than 12 ng/l and if the difference between the amount of the cardiac Troponin in the second sample and the amount in the first sample is at least 15 ng/l,
(ii) if the amount of the cardiac troponin in the first sample is larger than or equal to 12 ng/l and less than 60 ng/l and if the difference between amount of the cardiac troponin in the second sample and the amount of the cardiac troponin in the first sample is at least 15 ng/l, or
(iii) if the amount of the cardiac Troponin in the first sample is at least 60 ng/l.

2. The method of claim 1, wherein (C) the subject requires further monitoring in order to rule-in or rule-out myocardial infarction if neither the criteria set forth in (A), nor the criteria set forth in (B) are fulfilled.

3. The method of claim 1 or 2, wherein (C) the subject requires further monitoring in order to rule-in or rule-out myocardial infarction,
(i) if the amount of the cardiac troponin in the first sample is less than 12 ng/l, and if the difference between the amount of the cardiac troponin in the second sample and the amount of the cardiac troponin in the first sample is larger than or equal to 3 ng/l and less than 15 ng/l, or
(ii) if the amount of the cardiac troponin in the first sample is larger than or equal to 12 ng/l and less than 60 ng/l and if the difference between the amount of the cardiac troponin in the second sample and the amount of the cardiac troponin in the first sample is less than 15 ng/l.

4. A method for ruling-out myocardial infarction in a subject presenting with chest pain, comprising the steps of:
a) determining the amount of a cardiac troponin in a first sample from the subject obtained at presentation to a physician, and
b) determining the amount of a cardiac troponin in a second sample from said subject obtained within one hour after the first sample,
wherein myocardial infarction can be ruled-out in the subject,
(i) if the subject is 75 years old or older, and if the amount of the cardiac troponin in the second sample is less than 25 ng/l, or
(ii) if the subject is younger than 75 years and the amount of the cardiac Troponin in the first sample is less than 12 ng/l, and if the difference between the amount of the cardiac troponin in the second sample and the amount in first sample is less than 3 ng/l.

5. A method for ruling-in myocardial infarction in a subject presenting with chest pain, comprising the steps of:
a) determining the amount of a cardiac troponin in a first sample from the subject obtained at presentation to a physician, and
b) determining the amount of a cardiac troponin in a second sample from said subject obtained within one hour after the first sample,
wherein myocardial infarction can be ruled-in in the subject,
(i) if the amount of the cardiac Troponin in the first sample is less than 12 ng/l and if the difference between the amount of the cardiac Troponin in the second sample and the amount in the first sample is at least 15 ng/l,
(ii) if the amount of the cardiac troponin in the first sample is larger than or equal to 12 ng/l and less than 60 ng/l and if the difference between amount of the cardiac troponin in the second sample and the amount of the cardiac troponin in the first sample is at least 15 ng/l, or
(iii) if the amount of the cardiac Troponin in the first sample is at least 60 ng/l.

6. The method according to any one of claims 1 to 5, wherein the subject is human.

7. The method according to any one of claims 1 to 6, wherein the cardiac troponin is troponin T.

8. The method according to any one of claims 1 to 7, wherein the sample is a serum or plasma sample.

9. The method according to any one of claims 1 to 8, wherein the first sample obtained at presentation has been obtained not more than 12 hours after the onset or peak of chest pain.

10. The method according to any one of claims 1 to 9, wherein the first sample obtained at presentation has been obtained not more than 6 hours, or not more than 3 hours af ter onset or peak of chest pain.

11. The method according to any one of claims 1 to 10, wherein the subject does not have impaired renal function.

12. In vitro use of a cardiac troponin or of a detection agent which specifically binds to a cardiac troponin in a first and second sample from a subject presenting with chest pain for i) for ruling-in myocardial infarction in the subject, or ii) for ruling-out myocardial infarction in the subject, wherein the first sample has been obtained at presentation and wherein the second sample has been obtained within one hour after said first sample.

13. A device for diagnosing whether a subject who presents with chest pain suffers from myocardial infarction, or not, said device comprising:
a) an analyzing unit comprising a detection agent for a cardiac Troponin which allows for the determination of the amount of a cardiac Troponin in a first and second sample of a subject; and
b) an evaluation unit comprising a data processor, said data processor being capable of comparing the amount of the cardiac troponin in the first sample and the amount of the cardiac troponin in the second sample to reference amounts, and further being capable of assessing the difference between the amount of the cardiac troponin in the second sample and the amount of said cardiac troponin the first sample, wherein said data processor has implemented an algorithm for diagnosing myocardial infarction, and wherein the algorithm is the algorithm as defined in claim 1.
